# EUROPEAN PATENT SPECIFICATION

(11) **EP 2 010 158 B1**
(45) Date of publication and mention of the grant of the patent: **17.02.2016**
(21) Application number: 07718791.2
(22) Date of filing: 26.04.2007
(51) Int. Cl.: A61K 9/26, A61K 9/20, A61K 9/28, A61K 9/16

(54) **CONTROLLED RELEASE FORMULATIONS COMPRISING UNCOATED DISCRETE UNIT(S) AND AN EXTENDED RELEASE MATRIX**
FORMULIERUNGEN MIT KONTROLLIERTER FREISETZUNG AUS UNBESCHICHTETEN DISKRETEN EINHEITEN UND MATRIX MIT VERZÖGERTER FREISETZUNG
FORMULATIONS À LIBÉRATION CONTRÔLÉE QUI COMPRENNENT UNE OU PLUSIEURS UNITÉS DISCRÈTES NON ENROBÉES ET UNE MATRICE À LIBÉRATION RETARDÉE

(30) Priority: 26.04.2006 AU 2006902139; 07.03.2007 AU 2007901159
(43) Date of publication of application: 07.01.2009
(73) Proprietor: Alphapharm Pty Ltd., Queensland 4300 (AU)
(72) Inventor: KERAMIDAS, Panagiotis, Carindale, Queensland 4152 (AU); MOONEY, Brett, Antony, Mt Ommaney, Queensland 4074 (AU); FERGUSON, Phillip John, Browns Plains, Queensland 4118 (AU)
(74) Representative: Gillard, Richard Edward
(86) International application number: PCT/AU2007/000544
(87) International publication number: WO 2007/121537

(56) References cited:
- EP-A1- 1 064 937
- WO-A1-00/38686
- WO-A1-03/063834
- WO-A1-2005/099674
- WO-A1-2007/029081
- WO-A2-2004/038428
- WO-A2-2005/065661
- WO-A2-2006/121560
- US-A1- 2004 097 484
- US-A1- 2005 226 923
- US-A1- 2006 057 197
- US-B1- 6 475 521
- US-B2- 6 340 475

## Description

### Technical Field

This invention relates to modified-release pharmaceutical compositions, processes to prepare said compositions and uses of said compositions. More particularly, the invention relates to modified-release pharmaceutical compositions, processes to prepare said compositions and uses of said compositions wherein the active pharmaceutical ingredient is selected from antacids, anti-inflammatory substances (including but not limited to non-steroidal anti-inflammatory drugs, NSAIDs, vasodilators, coronary vasodilators, cerebral vasodilators, and peripheral vasodilators), anti-infectives, psychotropics, antimanics, stimulants, antihistamines, laxatives, decongestants, vitamins, gastrointestinal sedatives, antidiarrheal preparations, antianginal drugs, antiarrhythmics, antihypertensive drugs, vasoconstrictors and migraine treatments, anticoagulants and anti-thrombotic drugs, analgesics, anti-pyretics, hypnotics, sedatives, antiemetics, antinauseants, anticonvulsants, neuromuscular drugs, hyper- and hypoglycemic agents, thyroid and antithyroid preparations, diuretics, antispasmodics, uterine relaxants, mineral and nutritional additives, anti-obesity drugs, anabolic drugs, erythropoietic drugs, antiasthmatics, particularly zopiclone, zolpidem, galantamine, rosiglitazone, eszopiclone and metformin, pharmaceutically acceptable salts thereof, more particularly galantamine and most particularly galantamine hydrobromide.

### Background Art

Many active pharmaceutical ingredients (API's) when formulated as immediate release conventional dosage forms, such as tablets, capsules or pellets, generally require administration two or more times each day with concomitant peaks and troughs in the plasma level of the active ingredient. However for many disease states, it is advantageous to maintain the plasma level of the API at or near a steady state. Certain prior art compositions achieve this by utilising a biphasic release profile wherein after ingestion of the dosage form there is an initial release of the API and then a sustained release in order to maintain the plasma concentration. The number of daily administrations may thus often be reduced, from three or four to two, and from two administrations to one. Such a form has the additional possible benefit that plasma levels of the active ingredient are more constant than for immediate release forms, and so, fewer side effects may be observed than from excessively high peak levels just after dosing, and a better therapeutic cover is obtained. Hypnotic API's in particular, for example zolpidem, benefit from this biphasic release profile. An initial dose above the minimum therapeutic concentration is required quickly to send the individual to sleep. However immediate release formulations have the disadvantage that once the dose is released and a peak concentration level obtained the plasma concentration falls as per a typical immediate release plasma profile. Normally another dose of medication would be taken but of course when the condition being promoted is sleep taking another dose would negate the effect the hypnotic drug is trying to induce. Thus an ideal situation would be an initial immediate release of the drug followed by a sustained release to maintain the sleep state.

Many solutions have been forwarded to the problem of providing a biphasic release profile.

WO01/78725 relates to hypnotic pharmaceutical compositions made from pellets and exhibiting a modified release. Zolpidem or a pharmaceutically acceptable salt thereof is a typical hypnotic. The pellets are preferably spherical and exhibit a dissolution profile that includes 60% of the hypnotic agent being release from the pellet not earlier than 5 minutes from the start of a specified in vitro dissolution test. Although the modified release profile can include 50% of the hypnotic agent being released not earlier than 15 minutes after the start of the dissolution test, the pellet preferably does not contain a release rate controlling excipient or coating. Instead, microcrystalline cellulose and the active constitute the majority of the pellet, e.g. 90% or more. Indeed all the examples 1 - 8 disclose compositions containing API (various salts of zolpidem or zopiclone, microcrystalline cellulose and water). Spherical pellets are also made by a convenient method that is applicable to any pharmaceutically active agent. Further the disclosure teaches away from compositions comprising a disintegrating agent, stating that "...such agents are not required and are preferably excluded..... as such agents would interfere with the desired modified release dissolution profile"

WO95/28147 or its equivalent, US Patent No. 6,290,990, (BASF) relates to uncoated slow-release matrix pellets with a spherical or lenticular shape and uniform maximum diameters in the range from 0.5 to 4 mm, composed of a) 0.1 - 87% by weight of at least one biologically active compound, b) 5 - 50% by weight of at least one water-insoluble polymer, c) 5 - 45% by weight of at least one lipophilic component as plasticizer for polymer b, d) 3 - 40% by weight of a natural or semisynthetic gel former and e) 0 - 50% by weight of one or more conventional formulation aids.

WO00/33835 (Synthelabo) relates to controlled-release dosage forms of zolpidem or salts thereof adapted to release zolpidem over a predetermined time period, according to a biphasic profile of dissolution. The biphasic release is achieved by a first and second phase. Where the first phase is an immediate release phase and the second phase is a prolonged release phase.

EP0288138 relates to a controlled release pharmaceutical composition containing a number of spheroids, the spheroids containing a water-insoluble drug dispersed in a controlled release matrix. The matrix contains between 70% and 99.5% (by weight) of microcrystalline cellulose, between 0.5% and 4% (by weight) of a cellulose derivative and, optionally, up to 26% of a sugar or a sugar alcohol. The water insoluble drug must dissolve in water (pH 5) at 20°C to a concentration of less than 1.0mg ml⁻¹, preferably less than 0.5mg ml⁻¹. Preferred drugs are non-steroidal anti-inflammatory agents, especially fenprofen calcium, ibuprofen, ketoprofen, naproxen, diclofenac sodium, fenbufen, flurbiprofen, indomethacin, oxyphenbutazone, phenylbutazone or piroxicam.

GB2,264,639 relates to a composition comprising a water-soluble active ingredient, particularly hydromorphone hydrochloride, in the form of spheroids consisting of active ingredient and spheronising agent. The spheronising agent is preferably microcrystalline cellulose and hydroxypropyl methylcellulose may be included as binder. It is claimed that the uncoated spheroids provide for modified release, rather than normal release as expected without the need for a release-controlling film coating. However a biphasic release profile is not indicated.

WO2005051322 teaches a sustained-release carvedilol composition displaying a biphasic release profile which exhibits a first plasma concentration peak level and a first Tmax pulse within 1 - 4 hours of ingestion and a second plasma concentration peak level and second Tmax pulse within 5 - 8 hours after ingestion. In certain embodiments the composition is a capsule comprising carvedilol free base; or a solubility enhanced carvedilol salt, solvate or anhydrous forms; pellet or microparticle mixture of immediate release coated pellet populations and controlled release coated pellet populations of differing sizes.

US Patent No. 6,660,299 teaches a modified-release composition comprising amoxicillin in an immediate release phase and a slow release phase, wherein the ratio of amoxycillin in the immediate and slow release phase is from 3:1 to 1:3, and wherein the AUC value is at least 80% of that of the corresponding dosage of amoxycillin taken as an immediate release tablet or capsules, over the same dosage period.

US Patent Application 20060240107 teaches a solid dosage formulation having a core with a pharmacological agent dispersed in a first controlled-release matrix from which release of the agent is relatively slow and a coat formed over the core and having the agent dispersed in a second controlled-release matrix from which release of the agent is relatively fast. The dosage form is manufactured by admixing the API with the core matrix materials and compressing. The core is then coated in a separate procedure with a coat comprising the API mixed with polyvinyl acetate (PVA) and polyvinylpyrrolidinone (PVP) and optionally other excipients.

WO2004098572 teaches a mono-compartmental osmotic-controlled delivery system, useful for biphasic release of glipizide, comprises a core comprising a glipizide, a semipermeable membrane enclosing the core and at least one passageway. In the examples given, the core is first manufactured by a wet granulation and compression technique, the cores are then precoated and coated and an orifice drilled into each tablet.

WO94/06416 (Jagotec AG) describes multi-layered tablets comprising 500 mg of amoxycillin distributed equally between an immediate release and a slow release layer. Furthermore, WO95/20946 (SmithKline Beecham) describes inter alia a layered tablet comprising about 500 mg amoxycillin having a first layer which is an immediate release layer and a second layer which is a slow release layer, the ratio of amoxycillin between the two layers being about 1:2.6, as well as an intermediate barrier layer. Further bilayered tablets comprising clavulanic acid and amoxycillin are described in WO98/05305 (Quadrant Holdings Ltd). In such tablets, a first layer comprises amoxycillin and a second layer comprises clavulanate and the excipient trehalose, to stabilise the clavulanate component.

In addition, WO95/28148 (SmithKline Beecham) describes inter alia tablet formulations comprising amoxycillin and, optionally, clavulanate having a core comprising amoxycillin coated with a release retarding agent and surrounded by an outer casing layer of amoxycillin and potassium clavulanate. The release retarding agent is an enteric coating, so that there is an immediate release of the contents of the outer core, followed by a second phase from the core which is delayed until the core reaches the intestine. Furthermore, WO96/04908 (SmithKline Beecham) describes inter alia tablet formulations comprising amoxycillin in a matrix, for immediate release, and granules in a delayed release form comprising amoxycillin. Such granules are coated with an enteric coating, so release is delayed until the granules reach the intestine.

WO2006010640 discloses controlled release multilayer tablet comprising at least two layers, one active ingredient with high pH-dependent solubility, pH maintaining excipient and a matrix forming excipient to solve the problem of maintaining a constant plasma concentration over a prolonged period of time. Further disclosed is that the tablet can be prepared in two steps: different powders are first manufactured corresponding to the first type or the second type layer composition, as described above, and the compressed to form the multilayer tablet. Again manufacture of the tablets comprises a relatively complicated process.

Further examples include WO2006082809 and WO2005013935 which relate to oral dosage forms comprising pellets of a first composition and pellets of a second composition arranged to release the drug at differing release rates. Preferably the release profile is independent of pH. Thus the composition is complicated by the need to manufacture two separate compositions for one dosage form with all the additional costs associated therewith.

Galantamine is a well known acetylcholinesterase inhibitor which is active at nicotinic receptor sites and is capable of passing the blood-brain barrier in humans. It presents no severe side effects in therapeutically effective dosages. Galantamine hydrobromide is marketed by Janssen Pharmaceuticals as the product, Razadyne (also known as Reminyl) and is indicated for the symptomatic treatment of mild to moderately severe dementia of the Alzheimer type. Razadyne XL is the once daily extended release formulation approved in the USA on 22 December 2004. The formulation comprises extended release pellets in combination with some immediate release pellets in capsules.

A number of different pharmaceutical compositions containing galantamine, or a salt or analogue thereof, have been proposed including a fast-dissolving tablet formulations containing spray-dried lactose monohydrate and microcrystalline cellulose in a 75:25 ratio (U.S. Pat. No. 6,099,863).

EP1169024 A (Janssen) discloses a formulation wherein pregelatinized starch is used to prevent dose-dumping from a hydrophilic controlled-release formulation. The hydrophilic controlled release formulation comprises pregelatinized starch, one or more active ingredients, one or more viscous hydrophilic polymers and optionally pharmaceutically acceptable formulating agents. Preferred hydrophilic polymers include hydroxypropyl cellulose and hydroxypropyl methylcellulose.

WO0038686 (Janssen) relates to a controlled release formulation containing galantamine as the active ingredient, characterized in that it comprises particles comprising galantamine or a pharmaceutically acceptable acid addition salt thereof, a water soluble pharmaceutically acceptable ex[sigma]ipient and optionally other pharmaceutically acceptable excipients, said particles being coated by a release rate controlling membrane coating. Dosage forms comprising a therapeutically effective amount of said controlled release formulations can be administered orally to a patient once daily. In preferred dosage forms, part of the galantamine is present in an immediate release form, for example, as particles lacking a release rate controlling membrane coating, or as immediate release pellets, or as a topcoat on the controlled release formulation.

A sustained release formulation is disclosed in US 5231811 wherein the sustained release coating comprises a mixture of at least three polymers each of which has a different role depending on the pH at which is dissolves.

Further sustained release compositions are disclosed in: CA1326632 relating to oral treatment of Alzheimer's disease and comprise particles of galantamine coated with layers of polyvinyl pyrrolidone ; WO 2005/065661 relating to a sustained-release formulation defined by its dissolution profile; US 2004/0097484 relating to once-a-day formulations; and WO 2005/099674 relating to formulations using amphiphilic starch.

It can be seen from the above and further prior art disclosures that there are many examples of dosage forms exhibiting biphasic release profiles. However, many of the disclosed dosage forms comprise coated particles compressed to form a tablet (generally coated) which disintegrates in the stomach to make available the multitude of coated units, or capsules where all or a proportion of the contents (powder, pellets or spheroids) are coated to effect the delayed release profile, multilayer tablets or osmotic-controlled devices. Alternatively the prior art controlled-release formulations described above achieve their release profiles by provision of polymeric materials such as pregelatinized starch to prevent dose-dumping of the active ingredient upon ingestion. In the case of the coated prior art formulations, typically the coatings are pH dependent. It is this pH dependency that provides for the extended release profile. The prior art formulation comprising uncoated pellets (WO01/78725) further expressly discloses that the presence of a disintegrant is not recommended as this can deleteriously affect the modified release dissolution profile of the hypnotic API therein.

The prior art formulations are relatively complicated to manufacture generally involving multiple step processes. For example, although it is a well established technique, coating can cause problems when formulating certain API's such as interaction of the coating polymers or other ingredients present in the coat with the active ingredient or excipients in the tablet core. Furthermore, coating, whether it be of the tablet core, granules, pellets or mini-tablets is an additional process step which increases the cost and complexity of formulating a drug product.

There is thus a need for a composition that provides a controlled release of an API in such a manner that mimics the release profile of the prior art biphasic formulations or the prior art multi-unit or multilayered tablet compositions and further does not require multiple step manufacturing processes, specialised equipment and still further does not require coating to achieve the advantageous release profile. In particular, when treating certain conditions such as Alzheimer's disease, it is advantageous to maintain the plasma level of galantamine at or near a steady state. When employing fast-dissolving tablets, this requires administration at least twice a day with concomitant peaks and troughs in the plasma level of the active ingredient. Accordingly, there is a need for a prolonged release pharmaceutical composition containing galantamine.

The above references are incorporated by reference herein where appropriate for appropriate teachings of additional or alternative details, features and/or technical background.

### Summary of Invention

The present invention relates to a controlled-release formulation comprising a pharmaceutically effective amount of an API. The controlled-release formulations of the invention are designed to overcome the problems disclosed above with the prior art formulations. Further, formulations according to the present invention provide a matrix based extended-release system formulated into units such as pellets or mini-tablets that exhibit drug release profiles similar or equivalent to the prior art biphasic control-release systems.
Accordingly, there is provided in a first aspect of the invention a controlled-release formulation comprising 1 to 20 distinct and discrete units located in physical juxtaposition within a capsule to enable administration to a patient in need of treatment in a single dose, wherein each unit comprises:
(i) a unit dose of an active pharmaceutical ingredient or pharmaceutically acceptable salt thereof;
(ii) an extended-release agent comprising a matrix of one or more polymers; and, optionally
(iii) one or more pharmaceutically acceptable excipients, each unit being in the form of an uncoated pellet or mini-tablet,
wherein the sum of the unit doses constitutes a pharmaceutically effective amount of the active pharmaceutical ingredient.

Preferred embodiments comprise one or more pharmaceutically acceptable excipients. The extended-release agent is a matrix comprised of one or more polymers (s). In such embodiments the active ingredient is preferably evenly dispersed within the polymer matrix of the extended-release agent. Advantageously, this provides dosage forms with good uniformity of dose at each strength and the high dose per unit avoids problems such as partitioning which is typical in small dose tablets. In a particularly preferred embodiment the or each unit is uncoated.

According to a further embodiment of the invention there is provided a formulation wherein the or each unit independently has a diameter of between about 1 mm and 10 mm preferably the pellet diameter is about 3 mm to 5 mm, more preferably 3 mm or alternatively 5 mm. In particularly preferred embodiments the or each unit independently comprises about 1 mg to 50 mg of galantamine more preferably about 1 mg to 8 mg, particularly preferred is 4 mg or in an alternative embodiment 8 mg.

A further embodiment of the invention comprises a formulation wherein the or each unit independently has a diameter of about 5mm. A preferred embodiment of the invention comprises a formulation wherein the or each unit independently comprises about 4 mg to 10 mg of galantamine, preferably about 7 mg to 8 mg and ideally about 8 mg.

The invention provides a formulation comprising 1 to 20 units. Preferred embodiments comprise 1, 2, 3, 4, 5, 6, 12 or more units.

In certain embodiments there is provided a composition according to the invention wherein the or each unit comprises about 0.5 to about 30% by weight of API. Preferably the or each unit comprises about 1 to about 10% or 2 - 5% of API. Particularly preferred is a composition wherein the or each unit comprises about 2% API.

Further preferred embodiments are directed towards a composition according to the invention further comprising by weight one or more of about 0 - 5% of a glidant, 0 - 5% of a lubricant, 0 - 5% by weight of a binder/diluent, 0 - 10% disintegrant and 10-80% of a filler. A particularly preferred unit comprises 2 - 6% API, 10 - 50% Kollidon® SR, 0 - 4% Povidone, 0 - 5% anhydrous colloidal silica, 0 - 10% crospovidone, 20 - 70% microcrystalline cellulose and 0 - 5% magnesium stearate.

It is also envisaged that in embodiments comprising more than one unit, each unit may be comprised of different amounts of API or even different API's. Further it is envisaged that the units may comprise different excipients or different amounts of the same excipients.

In an embodiment the invention provides an extended release formulation comprising one or more unit(s) characterised in that the or each unit comprises about 1 - 10% of a pharmaceutically effective amount and about 10 - 90% of an extended-release agent and optionally comprises one or more further pharmaceutical ingredients. Preferably the extended release agent is a polymer matrix. Particularly preferred embodiments comprise polymer matrices comprising a copolymer of vinylpyrollidone and vinylacetate, preferably the mixture is of a type known as Kallidon^{®} SR available from BASF. Other embodiments of a capsule according to the invention comprise polyvinylpyrrolidone, such as Povidone K90, as a binder and polymer matrix extended-release agent. The provision of a polymer matrix according to the invention has been found to have advantageous consequences. In particular the inventors have found that such a matrix in combination with a disintegrant unexpectedly and advantageously mimics the biphasic release profile of the prior art Formulations.

Accordingly in another embodiment of a composition according to the invention there is provided a controlled- release composition as defined herein comprising, one or more uncoated unit(s), where the or each unit comprise(s):
(i) a pharmaceutically effective amount of an active pharmaceutical ingredient;
(ii) one or more extended-release agent(s); and
(iii) a disintegrant and optionally comprising one or more pharmaceutically acceptable excipients.

Hydrogenated vegetable oil may also be included in the formulations according to the invention to add to the extended-release dissolution profile of the formulation. This excipient is believed to act as a combined retard agent and lubricating agent. The amount of this excipient can be tailored to adjust the release profile of the formulation during dissolution testing to speed up or slow down the rate of the release of the API from the dosage form.

According to a further aspect of the invention there is provided a controlled release composition comprising an active pharmaceutical ingredient, a polymer matrix which is a copolymer of vinylpyrollidone and vinylacetate, hydrogenated vegetable oil and, optionally, further pharmaceutically acceptable excipients.

The matrix release profile is pH independent unlike the coated formulations disclosed in the prior art. Thus the formulations according to the invention can be manufactured using standard equipment and practices. There is no need for specialised technology. It has been noted by the formulators that the hardness of the matrix-type tablet is influential on the rate of release.

According to a further embodiment of the invention there is provided a method for preparing a controlled release pharmaceutical formulation as defined herein comprising the steps of:
(1) prepaying distinct and discrete units wherein the or each unit comprise(s):
   (i) a unit dose of an active pharmaceutical ingredient or pharmaceutically acceptable salt thereof;
   (ii) one or more extended-release agent (s) ; and, optionally,
   (iii) one or more pharmaceutically acceptable excipients; and
(2) bringing one or more said units into physical juxtaposition to enable administration in a single dose.

Further embodiments provide a formulation comprising excipients selected from one or more of: lubricant(s). filler(s), disintegrant(s) and/or binder(s). A further embodiment has magnesium stearate as the lubricant.

A particularly preferred embodiment of a formulation according to the invention provides an extended-release capsule as described wherein the or each unit comprise(s) galantamine HBr (10.256mg), Kollidon^{®} SR (46.744mg), hydrogenated vegetable oil (28mg), Povidone K90 (4mg) and magnesium stearate (1 mg).

The units are placed in a capsule shell. Accordingly capsules comprising units according to the invention are provided. Capsules comprising 1 to 20 unit(s), preferably 1 to 3 units, alternatively 6, 12 or 18 units.

Representative types of API's include antacids, anti-inflammatory substances, (including but not limited to non-steroidal anti-inflammatory drugs, NSAIDs, vasodilators, coronary vasodilators, cerebral vasodilators, and peripheral vasodilators), anti-infectives, psychotropics, antimanics, stimulants, antihistamines, laxatives, decongestants, vitamins, gastrointestinal sedatives, antidiarrheal preparations, antianginal drugs, antiarrhythmics, antihypertensive drugs, vasoconstrictors and migraine treatments, anticoagulants and anti-thrombotic drugs, analgesics, anti-pyretics, hypnotics, sedatives, antiemetics, antinauseants, anticonvulsants, neuromuscular drugs, hyper- and hypoglycemic agents, thyroid and antithyroid preparations, diuretics, antispasmodics, uterine relaxants, mineral and nutritional additives, anti-obesity drugs, anabolic drugs, erythropoietic drugs, antiasthmatics, particularly preferred API's include zopiclone, zolpidem, galantamine, rosiglitazone, eszopiclone and metformin, pharmaceutically acceptable salts thereof, more particularly galantamine and most particularly galantamine hydrobromide.

As used throughout this specification and the appended claims, the terms "sustained or extended release", "prolonged release", and "controlled release", as applied to drug compositions, have the meanings ascribed to them in "Remington's Pharmaceutical Sciences," 18th Ed., p.1677, Mack Pub. Co., Easton, Pa. (1990). Sustained or extended release drug systems include any drug delivery system which achieves the slow release of drug over an extended period of time, and include both prolonged and controlled release systems. If such a sustained release system is effective in maintaining substantially constant drug levels in the blood or target tissue, it is considered a controlled release drug delivery system. If, however, a drug delivery system is unsuccessful at achieving substantially constant blood or tissue drug levels, but nevertheless extends the duration of action of a drug over that achieved by conventional delivery, it is considered a prolonged release system. It will be understood that all embodiments within the scope of the invention are intended to display a biphasic-release dissolution profile.

According to a further aspect of the invention there is provided a controlled-release formulation, exhibiting a
dissolution profile across the physiological pH range, preferably in media comprising water, 0.1N HCl, pH 4.5 acetate buffer and pH 6.8 phosphate buffer for a candidate formulation, whereby
the dissolution profile as the a mount of active pharmaceutical ingredient dissolved for the candidate formulation in each medium is <25% in 30 minutes, 20-35% in 60 minutes, 35-55% in 180 minutes, 50-75% in 360 minutes and >65% in 720 minutes.

In an embodiment, the dissolution profile for the candidate formulation in each medium is <20% in 30 minutes, 25-30% in 60 minutes, 40-50%, preferably 45-50%, in 180 minutes, 60-70% in 360 minutes and >70%, preferably >80%, in 720 minutes.

In an embodiment, said media further comprise a surfactant, such as sodium lauryl sulfate, to increase the solubility of low solubility drugs, enzymes to mimic gastric fluid or other additives to mimic portions of the gastrointestinal tract.

According to a further aspect of the invention there is provided a controlled-release formulation exhibiting a dissolution profile in media comprising water, 0.1N HCl, pH 4.5 acetate buffer and pH 6.8 phosphate buffer wherein there is <25% dissolution in 30 minutes, 20-35% dissolution in 60 minutes, 35-55% dissolution in 180 minutes, 50-75% dissolution in 360 minutes and >65% dissolution in 720 minutes.

### Detailed Description of the Invention

The invention provides a controlled-release solid pharmaceutical formulation comprising one or more uncoated pellet(s) or mini-tablet(s) comprising a pharmaceutically effective amount of an API and an extended-release agent. The extended-release agent may suitably be selected from standard commercially available extended-release polymers known in the art, such as the following agents polyvinyl acetate (PVA) & polyvinylpyrrollidone (PVP) copolymer such as Kollidone^{®} SR, polyvinylpyrollidone such as Povidone K90, methacrylic/methacrylate polymers and copolymers such as Eudragit^{®} RL, Eudragit^{®} RS and Eudragit^{®} NE40, celluloses such as ethylcellulose and hydroxypropyl methylcellulose such as Hypromellose K100MCR. Kollidon^{®} SR was found by the inventor's to be a particularly suitable extended-release agent for this formulation. In a particularly preferred embodiment the composition of the uncoated pellets further comprises a disintegrant.

Preferably the ratio of extended release agent to API is about 1:1 to about 100:1, a particularly preferred embodiment comprises a ratio of between about 1:1 to 50:1, more preferably about 1:1 to 7:1. Particularly preferred is a ratio of about 1:3, 1:4.5 or 1:6.

Certain embodiments of a capsule according to the invention further comprise excipients that aid in the manufacture and stability of the capsules. In a preferred embodiment capsules according to the invention comprise a lubricant. The lubricant can be any type typically used in art of pharmaceutical formulations. The inventors found that magnesium stearate performed particularly well. The inventors have also found in certain preferred embodiments according to the invention that the inclusion of a disintegrant provides compositions according to the invention having particularly advantageous properties. This is surprising as similar compositions disclosed in what in the inventor's view is the closest prior art expressly state that a disintegrant is not necessary and in fact is detrimental to the release profile of the associated hypnotic drug.

Examples of disintegrants include Crospovidone, sodium starch glycollate and croscarmellose sodium type A but other ingredients that can act like a disintegrant are L-HPC, HPMC and other swelling polymers depending on the amounts. It will be understood by the skilled artisan that any disintegrant may be used in the exploitation of the invention, however particularly preferred is the use of Crospovidone, a commercially available disintegrant. It is also well within the skill-set of the skilled artisan to determine the amount of disintegrant needed without inventive faculty. However in certain embodiments of a controlled-release composition according to the invention approximately 1 - 15% by weight is preferred, particularly 2 - 10% most preferably 3 - 5%.

Examples of further excipients that can be utilised include lactose monohydrate as a diluent and silica colloidal anhydrous as a glidant. Other well known excipients can be used in the formulations of the invention for their common uses.

### Examples

The following examples are merely illustrative of the present invention and they should not be considered as limiting the scope of the invention

The following examples relate to a composition according to the invention. The units in the examples are mini-tablets and can be prepared by any means known in the art. A particularly preferred method is direct compression. The units according to the invention can be any size or shape such round, oval or oblong. Typically the units can range in size from about 1 mm to 10 mm, preferably from about 3 mm to 5 mm. The prepared mini-tablet(s) are then loaded into a capsule shell. The number of mini-tablets depends on the amount of active ingredient in each and the overall dosage required.

### Example 1

| Ingredient | mg/Tablet |
|---|---|
| Galantamine HBr | 10.256 |
| (equivalent to 8mg Galantamine) | |
| Povidone K90 | 4.0 |
| PVA & PVP | 46.744 |
| Hydrogenated Vegetable Oil | 28.0 |
| Magnesium Stearate | 1.0 |

The above example shows a capsule according to the invention. The ingredients were mixed and compressed into 3mm mini-tablets. To obtain the required dose, a capsule comprising 8mg of galantamine comprised six normal, round convex mini-tablets with each having a gross weight of approximately 15mg. A capsule comprising 16mg of galantamine comprises twelve mini-tablets and a capsule comprising 24mg comprises eighteen mini-tablets.

The following examples show a typical formulation of a capsule according to the invention wherein the mini-tablets are 5mm in diameter:

### Example 2

| Ingredient | mg/Tablet |
|---|---|
| Galantamine HBr | 10.256 |
| (equivalent to 8mg Galantamine) | |
| Povidone K90 | 2.0 |
| PVA & PVP | 23.244 |
| Hydrogenated Vegetable Oil | 14.0 |
| Magnesium Stearate | 0.5 |

The above example shows another embodiment of a capsule according to the invention. Again the ingredients were mixed and compressed, this time into 5mm mini-tablet. A capsule of 8mg of galantamine comprising one 5mm mini-tablet. Further embodiments include a capsule of 16mg of galantamine comprising two 5mm mini-tablets and a capsule of 24mg galantamine comprising three mini-tablets.

### Example 3

| Ingredient | mg/Tablet |
|---|---|
| Galantamine HBr | 10.256 |
| (equivalent to 8mg Galantamine) | |
| Povidone K90 | 2.0 |
| PVA & PVP | 26.244 |
| Hydrogenated Vegetable Oil | 11.0 |
| Magnesium Stearate | 0.5 |

The above example shows another embodiment of a capsule according to the invention. Again the ingredients were mixed and compressed, this time into 5mm mini-tablet. A capsule of 8mg of galantamine comprising one 5mm mini-tablet. Further embodiments include a capsule of 16mg of galantamine comprising two 5mm mini-tablets and a capsule of 24mg galantamine comprising three mini-tablets.

• The following example shows a capsule comprising coated units.

### Example 4

| Mini-tablets Ingredient | mg/Tablet |
|---|---|
| Galantamine HBr | 5.128 |
| (equivalent to 4mg Galantamine) | |
| Lactose (Spray dried) | 21.122 |
| Hypromellose K100MCR | 18.0 |
| Silica Colloidal Anhydrous | 0.25 |
| Magnesium Stearate | 0.5 |
| | |

| Coating | |
|---|---|
| Eudragit^{®} RL | 0.135 |
| Talc | 0.675 |
| Triethylcitrate | 0.225 |
| Eudragit^{®} RS | 1.215 |

The above example shows another embodiment of a capsule according to the invention. Again the ingredients were mixed and compressed, this time into 5mm mini-tablet. A capsule of 8mg of galantamine comprising one 5mm mini-tablet. Further embodiments include a capsule of 16mg of galantamine comprising two 5mm mini-tablets and a capsule of 24mg galantamine comprising three mini-tablets.

• The inventors have found that formulations according to the invention display a number of advantages. Firstly, as wet granulation is not used to prepare the units the production of impurities such as the N-oxide of galantamine is avoided. Secondly, the units according to the invention as exemplified above show excellent uniformity of dose at each strength and thirdly, the relatively high dose of galantamine per unit avoids problems such as partitioning, typical in small dose tablets.

Table 1 shows the dissolution profile of the various examples of formulations according to the present invention. It can be seen that the unit(s) provide a consistent release profile.

**TABLE 1**

| % Release | | | | |
|---|---|---|---|---|
| Time (mins) | Example 1 (pH 6.8 buffer) | Example 2 (pH 6.8 Buffer) Batch 1 | Example 2 (pH6.8 Buffer) Batch 2 | Example 3 (pH6.8 Buffer) |
| 0 | 0 | 0 | 0 | 0 |
| 15 | 12 | 8 | 11 | 12 |
| 30 | 18 | 15 | 16 | 18 |
| 45 | 21 | 20 | 19 | 23 |
| 60 | 24 | 24 | 22 | 26 |
| 90 | 29 | 31 | 27 | 32 |
| 120 | 34 | 37 | 32 | 37 |
| 180 | 42 | 47 | 39 | 44 |
| 240 | 49 | 56 | 46 | 51 |
| 300 | 56 | 63 | 51 | 57 |
| 360 | 61 | 69 | 56 | 62 |
| 420 | 65 | 74 | 60 | 66 |
| 480 | 69 | 77 | 64 | 70 |
| 1440 | 92 | 94 | 98 | 102 |

Table 2 shows that with formulations according to the invention, dissolution is not affected to a significant degree by changes in pH of the surrounding medium. Thus the formulations according to the invention can be manufactured using standard equipment and practices. There is no need for specialised technology.

**TABLE 2**

| % Release | | | |
|---|---|---|---|
| Time | pH 6.8 | pH 1-2 | pH 4.5 |
| 0 | 0 | 0 | 0 |
| 15 | 11 | 12 | 10 |
| 30 | 18 | 17 | 16 |
| 45 | 22 | 21 | 19 |
| 60 | 25 | 24 | 22 |
| 90 | 31 | 29 | 27 |
| 120 | 36 | 32 | 32 |
| 180 | 43 | 38 | 38 |
| 240 | 50 | 43 | 44 |
| 300 | 55 | 47 | 49 |
| 360 | 60 | 50 | 53 |
| 420 | 64 | 54 | 57 |
| 480 | 68 | 56 | 60 |
| 1440 | 102 | 86 | 93 |

### Example 5

There are a number of different media that can be used to "simulate" or mimic different conditions of the digestive tract. The main four that are commonly used are water, 0.1N HCl, pH4.5 Acetate buffer & pH6.8 Phosphate buffer. These equate to pH neutral for water, the stomach under fasted conditions (usually pHl-2) for the acid, the stomach under fed conditions (usually pH 3-7) for pH4.5 and either the stomach under fed conditions or lower parts of the GI tract (ie. small or large intestines) for the pH6.8. In order to establish appropriate bioavailability we have set out some limits for dissolution at various time points as set out in Table 3.

**TABLE 3**

| Time (mins) | Limit | Preferable | More preferable |
|---|---|---|---|
| 0 | 0 | 0 | 0 |
| 30 | <25% | <20% | |
| 60 | 20-35 | 25-30 | |
| 180 | 35-55 | 40-50 | 45-50 |
| 360 | 50-75 | 60-70 | |
| 720 | >65% | >70% | >80% |

### Example 6

Table 4 details a generalized formulation according to the invention. It is to be understood that it is not necessary for all the excipients from group B to be present in a composition according to the invention. One or more of these excipients may be present depending on the release profile desired.

**TABLE 4**

| Ingredient | % |
|---|---|
| A) | |
| Active Pharm Ingredient | 2-6 |
| Extended-release agent | ∼10-50 |

| B) | |
|---|---|
| Disintegrant | 1-15 |
| Binder | 3-5 |
| Glidants or flow agent | ≤ 2 |
| Filler | ∼20-70 |
| Lubricant | ∼1 |
| Total | 100 |

The active pharmaceutical ingredient according to the invention may comprise any of the following selected from the group comprising anti- arrhythmia's, anti-anginal, antacids, anti-inflammatory substances, (including but not limited to non-steroidal anti-inflammatory drugs, NSAIDs, vasodilators, coronary vasodilators, cerebral vasodilators, and peripheral vasodilators), anti-infectives, psychotropics, anti-manics, stimulants, antihistamines, laxatives, decongestants, vitamins, gastrointestinal sedatives, antidiarrheal preparations, anti-anginal drugs, antiarrhythmics, antihypertensive drugs, vasoconstrictors and migraine treatments, anticoagulants and anti-thrombotic drugs, analgesics, anti-pyretics, hypnotics, sedatives, antiemetics, antinauseants, anticonvulsants, neuromuscular drugs, hyper- and hypoglycemic agents, thyroid and antithyroid preparations, diuretics, antispasmodics, uterine relaxants, mineral and nutritional additives, anti-obesity drugs, anabolic drugs, erythropoietic drugs, antiasthmatics, particularly preferred API's include zopiclone, zolpidem, galantamine, rosiglitazone, eszopiclone and metformin, pharmaceutically acceptable salts thereof, more particularly galantamine and most particularly galantamine hydrobromide.

The extended-release agent may comprise any of a number of polymeric agents known in the art. These agents are generally in the form of soluble or insoluble polymer matrix systems. The soluble or hydrophilic colloid matrix systems generally comprise a compressed intimate mixture of API and one or more water-swellable hydrophilic polymer(s). Polymers include cellulose ethers such as methyl cellulose, ethyl cellulose or hydroxypropyl methylcellulose, sodium carboxymethyl cellulose, alginates, xanthan gum and carbopol. When compositions comprising said polymers are administered orally, the polymer(s) swell upon hydration from moisture in the digestive system, thereby limiting exposure of the active ingredient to moisture. A gel layer is formed which impedes further transgression of moisture into the composition core. The gel layer gradually dissolves or erodes allowing moisture to penetrate the gel matrix and the active ingredient slowly dissolves and diffuses through the gel, making it available for absorption by the body. An example of such an extended release dosage form of the analgesic/antiinflammatory drug etodolac (Lodine™) appears in US Patent 4,966,768. The insoluble polymer matrix systems comprise polymers that are not soluble in aqueous conditions such as the gut. Drug release rate from such systems depends on molecules in aqueous solution diffusing through a network of capillaries formed between compacted polymer particles.

The binder maybe any employed in the art to fulfil this function. Particularly preferred is Povidone^{®} K90.

The glidant preferably comprises silica colloidal anhydrous and/or talc.

Preferably the filler is a polymer or a combination of polymers. The inventors have found that microcrystalline cellulose is particularly preferred.

A preferred lubricant is magnesium stearate.

Of course it will be understood by those in the art that the above listed ingredients and their amounts may be altered within the limits of the claimed invention. Further lubricants may be chosen from depending on compatibility with other ingredients of the composition.

### Example 7

This example shows a particularly preferred embodiment according to the invention of a capsule comprising 1 x 5 mm mini-tablet in a capsule.

**TABLE 5**

| Ingredient | mg |
|---|---|
| Zolpidem | 1 |
| Kollidon SR | 11 |
| Povidone K30 | 2 |
| Colloidal Silica, Anhydrous | 0.5 |
| Microcrystalline Cellulose | 35 |
| 101 | |
| Magnesium Stearate | 0.5 |
| Total | 50 |

### Example 8

Shows a generalised embodiment according to the invention of a capsule comprising 1 x 5 mm mini-tablet in a capsule.

**TABLE 6**

| Ingredient | % |
|---|---|
| Rosiglitazone | 2-6 |
| Eudragit | -10-50 |
| Povidone K30 | 3-5 |
| Colloidal Silica, Anhydrous | ≤ 2 |
| Microcrystalline Cellulose | -20-70 |
| 101 | |
| Magnesium Stearate | -1 |
| Total | 100 |

With the above examples of a capsule according to the invention the ingredients were mixed and compressed into 1 x 5 mm mini-tablets, using standard techniques well known in the art.

The inventors have found that formulations according to the invention display a number of advantages. The units according to the invention as exemplified above show excellent uniformity of dose at each strength and the relatively high dose of API per unit avoids problems such as partitioning, typical in small dose tablets.

## Claims

1. A controlled-release formulation comprising 1 to 20 distinct and discrete units located in physical juxtaposition within a capsule to enable administration to a patient in need of treatment in a single dose, wherein each unit comprises:
(i) a unit dose of an active pharmaceutical ingredient or pharmaceutically acceptable salt thereof;
(ii) an extended-release agent comprising a matrix of one or more polymers; and, optionally
(iii) one or more pharmaceutically acceptable excipients,
each unit being in the form of an uncoated pellet or mini-tablet,
wherein the sum of the unit doses constitutes a pharmaceutically effective amount of the active pharmaceutical ingredient.

2. The formulation as claimed in claim 1 wherein each unit has a diameter of between 1mm and about 10mm.

3. The formulation as claimed in claim 2 wherein each unit has a diameter of about 3mm, preferably of about 5mm.

4. The formulation as claimed in claim 1 comprising 2 to 20 units.

5. The formulation as claimed in claim 1 wherein the extended-release agent comprises a blend of polyvinylpyrrolidone and polyvinylacetate.

6. The formulation as claimed in any one of claims 1 to 5 wherein the extended-release agent further comprises a disintegrant.

7. The formulation as claimed in any one of claims 1 to 6 wherein the extended-release agent additionally comprises a combined retard and lubricating agent.

8. The formulation as claimed in claim 7 wherein the combined retard and lubricating agent is hydrogenated vegetable oil

9. The formulation as claimed in anyone of claims 1 to 8 wherein the active pharmaceutical ingredient is selected from the group consisting of antacids, non-steroidal anti-inflammatory drugs (NSAIDs), vasodilators, coronary vasodilators, cerebral vasodilators, peripheral vasodilators, anti-infectives, psychotropics, antimanics, stimulants, antihistamines, laxatives, decongestants, vitamins, gastrointestinal sedatives, antidiarrhealpreparations, antianginal drugs, antiarrhythmics, anti-hypertensive drugs, vasocontrictors and migraine treatments, anticoagulants and anti-thrombotic drugs, analgesics, anti-pyretics, anticonvulsants, neuromusculardrugs, hyper- and hypoglycaemic agents, thyroid andantithyroid preparations, diuretics, antispasmodics, uterine relaxants, mineral and nutritional additives, anti-obesity drugs, anabolic drugs, erythropoietic drugs and antiasthmatics.

10. A formulation as claimed in any one of claims 1 to 9 which is a tablet formulation comprising a matrix of one or more pharmaceutically acceptable excipients and a plurality of units comprising a predetermined amount of an active pharmaceutical ingredient and one or more extended release agents, the plurality of units dispersed within said matrix.

11. A formulation as claimed in any one of claims 1 to 10, exhibiting a dissolution profile in media comprising water, 0.1N HCl, pH 4.5 acetate buffer and pH 6.8 phosphate buffer, wherein the amount of active pharmaceutical ingredient dissolved in each medium is <25% in 30 minutes, 20-35% in 60 minutes, 35-55% in 180 minutes, 50-75% in 360 minutes and >65% in 720 minutes.

12. The formulation as claimed in claim 11 wherein the dissolution profile in each medium is <20% in 30 minutes, 25-30% in 60 minutes, 40-50%, preferably 45-50%, in 180 minutes, 60-70% in 360 minutes and >70%, preferably >80%, in 720 minutes.

13. The formulation as claimed in claim 1 wherein the active pharmaceutical ingredient is galantamine or a pharmaceutically acceptable salt thereof.

14. The formulation as claimed in claim 13 wherein the unit dose of galantamine is 8 mg.

15. The formulation of claim 14 consisting of 1, 2 or 3 units, wherein each unit comprises:
(i) a unit dose of 8 mg of galantamine;
(ii) a polymer matrix which is a mixture of polyvinylpyrrolidone and polyvinylacetate;
(iii) hydrogenated vegetable oil;
(iv) povidone; and
(v) magnesium stearate.

## Patentansprüche

1. Formulierung zur kontrollierten Freisetzung, umfassend 1 bis 20 unterschiedliche und eigenständige Einheiten, die in einer physikalischen Juxtaposition innerhalb einer Kapsel angeordnet sind, um die Verabreichung an einen Patienten, welcher der Behandlung bedarf, in einer Einzeldosis zu ermöglichen, wobei jede Einheit Folgendes umfasst:
(i) eine Dosiseinheit eines aktiven pharmazeutischen Inhaltsstoffes oder pharmazeutisch verträglichen Salzes davon;
(ii) ein Mittel zur verzögerten Freisetzung, umfassend eine Matrix aus einem oder mehreren Polymeren; und, optional,
(iii) einen oder mehrere pharmazeutisch verträgliche Hilfsstoffe,
wobei jede Einheit in der Form eines unbeschichteten Pellets oder einer Minitablette vorliegt,
wobei die Summe der Dosiseinheiten eine pharmazeutisch wirksame Menge des aktiven pharmazeutischen Inhaltsstoffes darstellt.

2. Formulierung nach Anspruch 1, wobei jede Einheit einen Durchmesser von 1 mm bis etwa 10 mm aufweist.

3. Formulierung nach Anspruch 2, wobei jede Einheit einen Durchmesser von etwa 3 mm aufweist, bevorzugt von etwa 5 mm.

4. Formulierung nach Anspruch 1, umfassend 2 bis 20 Einheiten.

5. Formulierung nach Anspruch 1, wobei das Mittel zur verzögerten Freisetzung eine Mischung von Polyvinylpyrrolidon und Polyvinylacetat umfasst.

6. Formulierung nach einem der Ansprüche 1 bis 5, wobei das Mittel zur verzögerten Freisetzung ferner ein Disintegrationsmittel umfasst.

7. Formulierung nach einem der Ansprüche 1 bis 6, wobei das Mittel zur verzögerten Freisetzung zusätzlich ein kombiniertes Verzögerungs- und Schmiermittel umfasst.

8. Formulierung nach Anspruch 7, wobei das kombinierte Verzögerungs- und Schmiermittel ein gehärtetes pflanzliches Öl ist.

9. Formulierung nach einem der Ansprüche 1 bis 8, wobei der aktive pharmazeutische Inhaltsstoff ausgewählt ist aus der Gruppe bestehend aus Säureblockern, nichtsteroidalen Entzündungshemmern (NSAIDs), Vasodilatatoren, Koronar-Vasodilatatoren, Zerebralvasodilatatoren, Peripherievasodilatatoren, Antünfektiosa, Psychopharmaka, Mittel gegen Manie, Stimulantien, Antihistamine, Abführmittel, Dekongestiva, Vitamine, gastrointestinale Sedativa, Antidiarrhoika, antianginöse Mittel, Antiarrhythmika, Mittel gegen Bluthochdruck, Vasokonstriktoren und Migränebehandlungen, Antikoagulantien und antithrombotische Mittel, Analgetika, Antipyretika, krampflösende Mittel, neuromuskuläre Mittel, hyper- und hypogylkämische Mittel, thyroidale und antithyroidale Präparate, Diuretika, Antispasmodika, Uterusrelaxantien, mineralische Zusatzstoffe und Nahrungsmittelzusatzstoffe, Mittel gegen Fettleibigkeit, anabolische Mittel, erythropoetische Mittel und Antiasthmatika.

10. Formulierung nach einem der Ansprüche 1 bis 9, die eine Tablettenformulierung ist, die eine Matrix aus einem oder mehreren pharmazeutisch verträglichen Hilfsstoffen und eine Vielzahl an Einheiten umfasst, die eine vorbestimmte Menge eines aktiven pharmazeutischen Inhaltsstoffes und eines oder mehrere Mittel zur verzögerten Freisetzung umfassen, wobei die Vielzahl der Einheiten in der Matrix dispergiert sind.

11. Formulierung nach einem der Ansprüche 1 bis 10, die ein Auflösungsprofil in Medien, die Wasser, 0,1 N HCL, pH 4,5-Acetatpuffer, und pH 6,8-Phosphatpuffer umfassen, wobei die Menge an aktivem pharmazeutischem Inhaltsstoff, die in jedem Medium aufgelöst wird, nach 30 Minuten <25 %, nach 60 Minuten 20-35 %, nach 180 Minuten 35-55 %, nach 360 Minuten 50-75 % und nach 720 Minuten >65 % beträgt.

12. Formulierung nach Anspruch 11, wobei das Auflösungsprofil in jedem Medium nach 30 Minuten <20 %, nach 60 Minuten 25-30 %, nach 180 Minuten 40-50 %, bevorzugt 45-50 %, nach 360 Minuten 60-70 %, und nach 720 Minuten >70 %, bevorzugt >80 % beträgt.

13. Formulierung nach Anspruch 1, wobei der aktive pharmazeutische Inhaltsstoff Galantamin oder ein pharmazeutisch verträgliches Salz davon ist.

14. Formulierung nach Anspruch 13, wobei die Dosiseinheit von Galantamin 8 mg beträgt.

15. Formulierung nach Anspruch 14, bestehend aus 1, 2 oder 3 Einheiten, wobei jede Einheit Folgendes umfasst:
(i) Eine Dosiseinheit von 8 mg Galantamin;
(ii) eine Polymermatrix, die eine Mischung aus Polyvinylpyrrolidon und Polyvinylacetat ist;
(iii) gehärtetes pflanzliches Öl;
(iv) Povidon; und
(v) Magnesiumstearat.

## Revendications

1. Formulation à libération contrôlée comprenant 1 à 20 unités distinctes et discrètes situées en juxtaposition physique au sein d'une gélule pour permettre l'administration à un patient nécessitant un traitement en dose unique, dans laquelle chaque unité comprend :
(i) une dose unitaire d'un ingrédient pharmaceutique actif ou d'un sel de celui-ci acceptable sur le plan pharmaceutique ;
(ii) un agent de libération prolongée comprenant une matrice d'un ou plusieurs polymères ; et, éventuellement,
(iii) un ou plusieurs excipients acceptables sur le plan pharmaceutique,
chaque unité étant sous la forme d'une pastille ou d'un mini-comprimé non revêtu,
dans laquelle la somme des doses unitaires représente une quantité efficace sur le plan pharmaceutique de l'ingrédient pharmaceutique actif.

2. Formulation selon la revendication 1, dans laquelle chaque unité a un diamètre compris entre 1 mm et environ 10 mm.

3. Formulation selon la revendication 2, dans laquelle chaque unité a un diamètre d'environ 3 mm, de préférence d'environ 5 mm.

4. Formulation selon la revendication 1, comprenant 2 à 20 unités.

5. Formulation selon la revendication 1, dans laquelle l'agent de libération prolongée comprend un mélange de polyvinylpyrrolidone et d'acétate de polyvinyle.

6. Formulation selon l'une quelconque des revendications 1 à 5, dans laquelle l'agent de libération prolongée comprend en outre un agent délitant.

7. Formulation selon l'une quelconque des revendications 1 à 6, dans laquelle l'agent de libération prolongée comprend en outre un agent combiné de retard et lubrifiant.

8. Formulation selon la revendication 7, dans laquelle l'agent combiné de retard et lubrifiant est de l'huile végétale hydrogénée.

9. Formulation selon l'une quelconque des revendications 1 à 8, dans laquelle l'ingrédient pharmaceutique actif est choisi dans le groupe constitué d'antiacides, médicaments anti-inflammatoires non stéroïdiens (AINS), vasodilatateurs, vasodilatateurs coronaires, vasodilatateurs cérébraux, vasodilatateurs périphériques, agents anti-infectieux, agents psychotropes, agents antimaniaques, stimulants, antihistaminiques, laxatifs, décongestionnants, vitamines, sédatifs gastro-intestinaux, préparations antidiarrhéiques, médicaments antiangineux, antiarythmiques, médicaments antihypertenseurs, vasoconstricteurs et traitements contre la migraine, médicaments anticoagulants et antithrombotiques, analgésiques, antipyrétiques, anticonvulsifs, médicaments neuromusculaires, agents hyper- et hypo-glycémiques, préparations thyroïdiennes et antithyroïdiennes, diurétiques, antispasmodiques, relaxants utérins, additifs minéraux et nutritionnels, médicaments antiobésité, médicaments anabolisants, médicaments érythropoïétiques et antiasthmatiques.

10. Formulation selon l'une quelconque des revendications 1 à 9, qui est une formulation en comprimé comprenant une matrice d'un ou plusieurs excipients acceptables sur le plan pharmaceutique et une pluralité d'unités comprenant une quantité prédéterminée d'un ingrédient pharmaceutique actif et d'un ou plusieurs agents de libération prolongée, la pluralité d'unités étant dispersée au sein de ladite matrice.

11. Formulation selon l'une quelconque des revendications 1 à 10, présentant un profil de dissolution dans des milieux comprenant de l'eau, du HCl 0,1 N, un tampon acétate à pH 4,5 et un tampon phosphate à pH 6,8, dans laquelle la quantité d'ingrédient pharmaceutique actif dissous dans chaque milieu est <25 % en 30 minutes, 20 à 35 % en 60 minutes, 35 à 55 % en 180 minutes, 50 à 75 % en 360 minutes et >65 % en 720 minutes.

12. Formulation selon la revendication 11, dans laquelle le profil de dissolution dans chaque milieu est <20 % en 30 minutes, 25 à 30 % en 60 minutes, 40 à 50 %, de préférence, 45 à 50 %, en 180 minutes, 60 à 70 % en 360 minutes et >70 %, de préférence >80 %, en 720 minutes.

13. Formulation selon la revendication 1, dans laquelle l'ingrédient pharmaceutique actif est la galantamine ou un sel de celle-ci acceptable sur le plan pharmaceutique.

14. Formulation selon la revendication 13, dans laquelle la dose unitaire de galantamine est de 8 mg.

15. Formulation selon la revendication 14, constituée de 1, 2 ou 3 unités, dans laquelle chaque unité comprend :
(i) une dose unitaire de 8 mg de galantamine ;
(ii) une matrice polymère qui est un mélange de polyvinylpyrrolidone et d'acétate de polyvinyle ;
(iii) une huile végétale hydrogénée ;
(iv) de la povidone ; et
(v) du stéarate de magnésium.
